# EUROPEAN PATENT SPECIFICATION

(11) **EP 4 549 953 B1**
(45) Date of publication and mention of the grant of the patent: **02.12.2026**
(21) Application number: 24209218.7
(22) Date of filing: 28.10.2024
(51) Int. Cl.: G01N 35/04, G01N 33/487, G01N 33/493

(54) **URINE TEST DEVICE**
URINTESTVORRICHTUNG
DISPOSITIF DE TEST D'URINE

(30) Priority: 30.10.2023 JP 2023186018; 18.10.2024 JP 2024184229
(43) Date of publication of application: 07.05.2025
(60) Divisional application: 26208224.1 / 4 838 218
(73) Proprietor: ARKRAY, Inc., Kyoto-shi, Kyoto 601-8045 (JP)
(72) Inventor: KOBAYASHI, Atsushi, Kyoto-shi, 602-0008 (JP); FURUSATO, Noriaki, Kyoto-shi, 602-0008 (JP)
(74) Representative: MFG Patentanwälte Meyer-Wildhagen Meggle-Freund Gerhard PartG mbB

(56) References cited:
- WO-A1-2011/122562
- US-A1- 2023 152 299
- US-B1- 8 535 618

## Description

### TECHNICAL FIELD

The present disclosure relates to a urine test device.

### BACKGROUND ART

A urine test device is a device that tests a sample by causing a reagent part provided on a surface of a test paper (also referred to as a test piece or the like) to develop a color by a reaction between the reagent part and urine as the sample, and detects the presence or absence, a degree, or the like of the color development. In this test, for example, physical properties of urine, or the presence or absence or concentration of a specific component in the urine is measured. In the related art, as described in Japanese Patent Application Laid-Open Publication No. H05-264540 (hereinafter, referred to as Patent Literature 1), a plurality of test papers are accommodated in a cylindrical rotatable drum, and one test paper is discharged from a discharge port of the drum. One test paper discharged (supplied) from the drum is conveyed by a sorting rack mechanism.

On the other hand, the reagent part may deteriorate due to the reagent part on the test paper absorbing moisture in an atmosphere (that is, moisture absorption). As described in Japanese Patent Application Laid-Open Publication No. H05-5736 (hereinafter, referred to as Patent Literature 2) and 2023-72560 (hereinafter, referred to as Patent Literature 3), various measures against deterioration of the reagent part have been studied.

In a device described in Patent Literature 2, a deterioration detection reagent part provided on the test paper is irradiated with light, and the degree of deterioration is determined based on the reflected light. In a device described in Patent Literature 3, a plurality of test papers are accommodated in a cylindrical container. One test paper is discharged through a rotatable door member attached to a side portion of the container. In a mechanism including the door member, two openings communicating with the inside of the container are not simultaneously opened, and a state in which one of the openings is normally closed is maintained. Accordingly, outside air is prevented from entering the container. US 2023/0152299 A1 discloses a test strip container including an accommodating member in which a test strip is accommodated, a moving member moving the test strip therein, a door member opening and closing at a side surface of the accommodating member to discharge the test strip out of the accommodating member, and cutting the interior and the exterior of the accommodating member off from each other when closed and a door accommodating portion covering the door member, in which the door member can open and close. WO 2011/122562 A1 discloses an analyzing device which is provided with a housing that houses the analyzing device, and a container disposed at a predetermined position in the housing. The analyzing device is also provided with a transfer mechanism which, by being associated with the operation performed by the operating section provided in the housing, transfers the container from the predetermined position to a position where the container is to be taken out. US 8535618 B1 discloses a test strip dispenser that has a housing that defines a dispensing opening. A cylindrical body engages the housing and is capable of rotating about an axis to rotate relative to the housing. A test strip engages an outer surface of the cylindrical body and is carried by the cylindrical body about the axis. Alignment of the test strip with the dispensing opening causes the test strip to fall off of and out of engagement with the outer surface and through the dispensing opening.

As described above, in the related art, measures against deterioration of the reagent part have been studied, but in any measure, it is necessary to prepare a special test paper or to incorporate a special mechanism or member into the device. Such measures may complicate the device or may increase the cost.

The present disclosure describes a urine test device capable of preventing, with a simple configuration, deterioration of a reagent part due to moisture in a test paper.

### SUMMARY OF INVENTION

According to a first solution, the present invention provides a urine test device in accordance with independent claim 1. Further aspects of the invention are set forth in the dependent claims, the drawings and the following description. An aspect of the present disclosure relates to a urine test device including: a conveying mechanism configured to convey a test paper provided with a reagent unit; a supply mechanism that includes an accommodating chamber capable of accommodating a plurality of test papers, a take-out unit configured to take out a test paper from the accommodating chamber and a discharge unit configured to discharge the test paper, and that is configured to sequentially supply the test paper to the conveying mechanism by discharging the test paper from the discharge unit; the conveying mechanism configured to receive the test paper from the supply mechanism, and convey the test paper to a deposition position to which a sample is deposited or a vicinity thereof; and a cover configured to cover the accommodating chamber and the discharge unit, and seal the take-out unit against outside air.

According to the urine test device described above, while the supply mechanism is operating, the test papers are discharged from the discharge unit. The test paper is conveyed to the deposition position or the vicinity thereof by the conveying mechanism. After the sample (that is, urine) is deposited on the reagent part of the test paper, a test relating to the sample is performed. When the supply mechanism is idle (namely, not operating or not working), for example, when the device is not operated for a while after the test of a large number of samples is finished, the discharge unit can be covered with the cover. Since the cover seals the accommodating chamber and the take-out unit against the outside air, even in a state where one or a large number of test papers are accommodated in the accommodating chamber, it is possible to prevent moisture from entering and prevent deterioration of the reagent part. Since the cover may be designed in accordance with a shape of the discharge unit or the like, large modification or the like of an existing device is unnecessary. Thus, according to the urine test device, it is possible to prevent, with a simple configuration, deterioration of the reagent part due to moisture in the test paper. In the case of a device that does not include a cover in the related art, in order to prevent deterioration of the reagent part due to moisture, it is necessary to take measures such as taking out all the test papers remaining in the accommodating chamber and separately storing the test papers. In this urine test device, since the cover seals the inside of the supply mechanism, such special measures are unnecessary.

The cover may be in close contact with the discharge unit by being sandwiched between the discharge unit and a part of the conveying mechanism, when the supply mechanism is idle. There is originally a space between the discharge unit and the conveying mechanism. By disposing the cover in this space and using a pressing force received from the discharge unit and the conveying mechanism, it becomes unnecessary to prepare a separate pressing mechanism or the like in close contact (sealing of the supply mechanism) with the discharge unit.

The urine test device may further include: a device main body including the conveying mechanism. The supply mechanism may be attached to the device main body and may be movable between an operating position where the discharge unit faces the conveying mechanism and a maintenance position where the discharge unit is separated from the conveying mechanism compared to the operating position, and the cover may be attached to the discharge unit when the supply mechanism is located at the maintenance position. When the supply mechanism is idle (when the test is finished or the like), the supply mechanism is often moved to the maintenance position. According to this configuration, the cover can be attached using an opportunity or time of the maintenance work. Further, even when the maintenance work is not performed, the discharge unit can be exposed only by moving the supply mechanism, and the cover can be easily attached.

The cover may be temporarily held by the discharge unit when the supply mechanism is located at the maintenance position, and may be fixed in close contact with the discharge unit along with a setting operation in which the supply mechanism is moved to the operating position and set in the device main body. According to this configuration, since the cover is brought into close contact and fixed in accordance with the setting operation of the supply mechanism, it is possible to reliably and easily attach the cover.

The supply mechanism may include a shutter attached in the vicinity of the discharge unit and configured to open the discharge unit only at the time of discharging of the one test paper and cover the discharge unit at a time other than the time of discharging, and the cover may be attached to the discharge unit using the shutter. According to this configuration, since the cover is attached using the existing shutter, a dedicated fixture, an attachment bracket, and the like are unnecessary. The above-described functions and effects are realized with a simpler configuration.

The device main body may be provided with a controller configured to control an operation of the urine test device, and the controller may perform operation prohibition control to prohibit the operation of the urine test device when detecting the cover being attached to the discharge unit. The operation prohibition control can prevent the supply mechanism and the first conveying mechanism from operating while the cover is erroneously attached.

According to the present disclosure, it is possible to prevent, with a simple configuration, deterioration of the reagent part due to moisture in the test paper.

### BRIEF DESCRIPTION OF DRAWINGS

Exemplary embodiment(s) of the present invention will be described in detail based on the following figures, wherein
Fig. 1 is a perspective view showing a urine test device according to an embodiment of the present disclosure;
Fig. 2 is a front view showing an internal schematic configuration of a device main body in Fig. 1;
Fig. 3 is a perspective cross-sectional view showing first and second conveying mechanisms, a deposition device, an imaging device, and a disposal box provided in the device main body;
Fig. 4 is a perspective view showing a supply mechanism and the first conveying mechanism located at a maintenance position;
Fig. 5 is a cross-sectional view showing the supply mechanism;
Fig. 6 is a perspective view of a cover as viewed from above (from a side in contact with a discharge unit);
Fig. 7 is a perspective view of the cover as viewed from below (from a side in contact with the first conveying mechanism);
Fig. 8 is a perspective view showing a shutter that covers the discharge unit;
Fig. 9A is a diagram showing a state where the shutter is opened to attach the cover;
Fig. 9B is a diagram showing a state where the cover is temporarily held by the discharge unit using the shutter; and
Fig. 10 is a cross-sectional view of the cover in close contact with the discharge unit when the supply mechanism is idle.

### DESCRIPTION OF EMBODIMENTS

Hereinafter, embodiments of the present invention will be described with reference to the drawings. The same elements are denoted by the same reference numerals, and redundant descriptions thereof will be omitted. The dimensional ratios in the drawings do not necessarily coincide with those in the description.

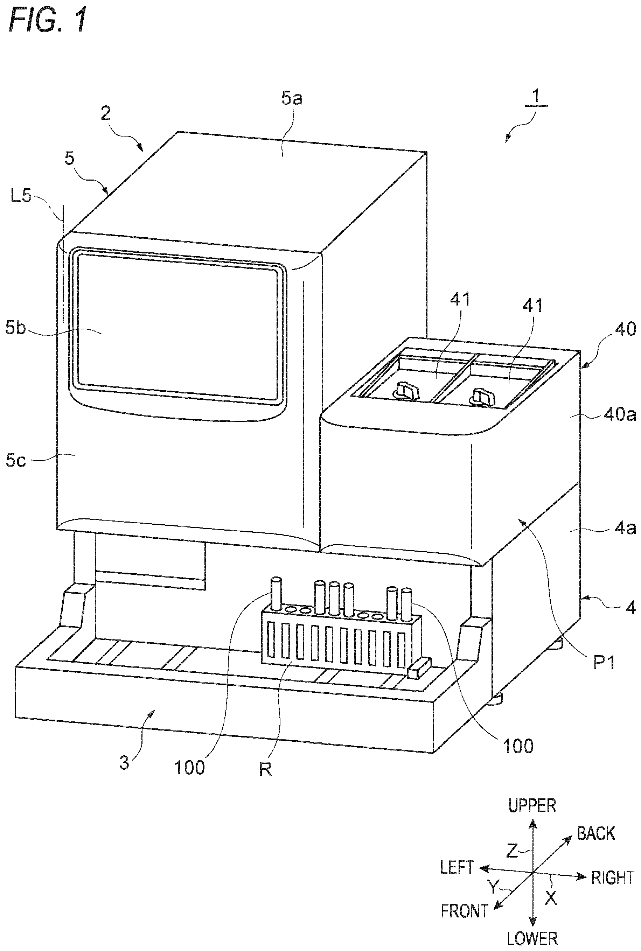

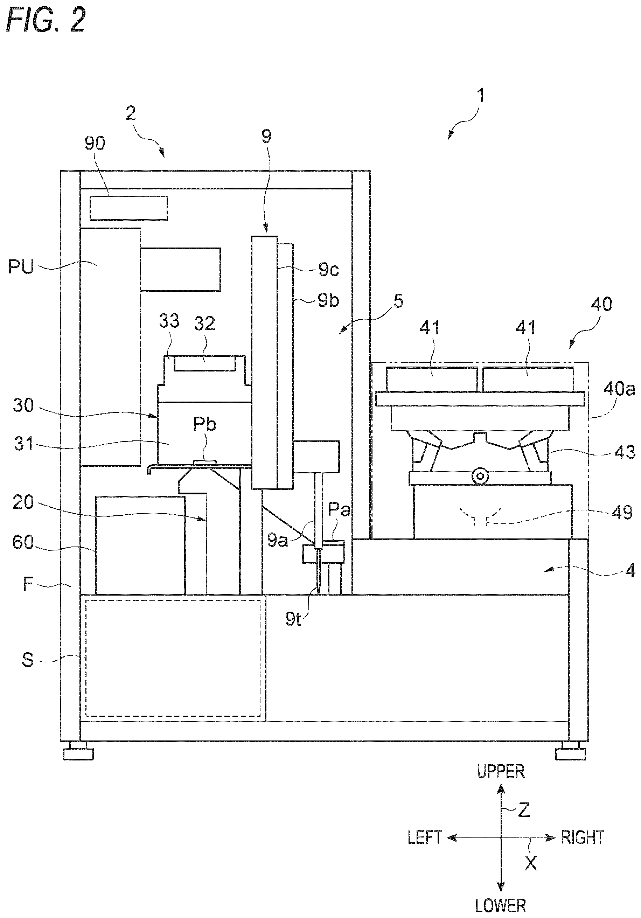

In some of the drawings, X-direction, Y-direction, and Z-direction orthogonal to each other are shown, and each of these directions may be used to describe a urine test device 1. X-direction, Y-direction, and Z-direction respectively correspond to a left-right direction, a front-rear direction, and an up-down direction in an installation state of the urine test device 1 shown in Figs. 1 and 2. In the present specification, terms indicating directions such as "front", "rear", "up", "down", "left", and "right" are terms based on an installation state of the urine test device 1. The urine test device 1 is installed on a flat and horizontal surface. "Left" and "right" are directions when the urine test device 1 is viewed from an operator who stands in front of a placement table 3 and a display 5b and faces the placement table 3 and the display 5b.

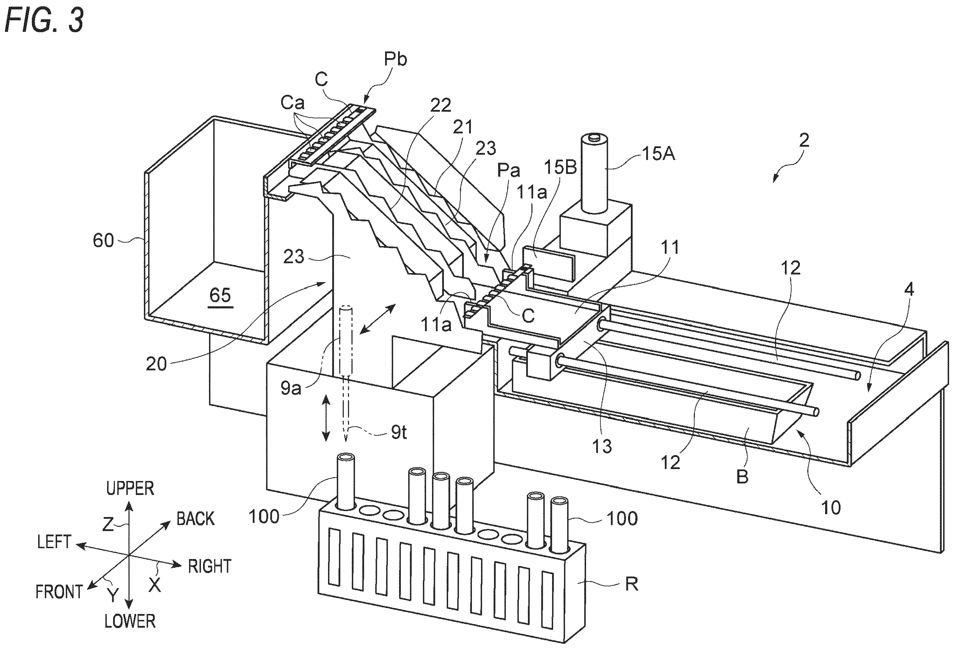

First, an overall configuration of the urine test device 1 will be described with reference to Figs. 1 and 2. The urine test device 1 is a device for testing urine as a sample accommodated in a spit 100. As shown in Fig. 1, the urine test device 1 sequentially tests samples in a plurality of spits 100 standing on a rack R. Test items (test purposes) are not particularly limited. Examples of the test items include physical properties of the sample or the presence or absence or concentration of a specific component in the sample. In the urine test device 1, an elongated test paper C is used for the test of the sample (see Fig. 3). One test paper C is used for one sample. Each test paper C is provided with, for example, a plurality of reagent parts Ca. Each reagent part Ca may correspond to different test items. Each reagent part Ca includes a reagent corresponding to a test item. A known configuration may be adopted as the test paper C.

As shown in Fig. 1, the urine test device 1 includes a device main body 2 in which various devices and mechanisms related to the conveyance of the test paper C and the test of the sample are accommodated, and the placement table 3 including a conveying mechanism and the like related to the supply and conveyance of the rack R. The device main body 2 includes a device lower portion 4 and a device upper portion 5 disposed on a portion (for example, a left side) of the device lower portion 4 in the left-right direction. The placement table 3 is coupled to a front surface of the device lower portion 4. The urine test device 1 further includes a supply mechanism 40, which is a mechanism for accommodating the test paper C and supplying the test paper C to the device main body 2. The supply mechanism 40 is disposed on another portion (for example, a right side) of the device lower portion 4 in the left-right direction.

As shown in Fig. 2, the device main body 2 includes a frame F formed of structural members that extend in the up-down direction, the left-right direction, and the front-rear direction. As shown in Fig. 1, the device lower portion 4 includes a lower housing 4a, and the device upper portion 5 includes an upper housing 5a. The lower housing 4a and the upper housing 5a form a rectangular parallelepiped shape based on a shape of the frame F. A front panel 5c that can be opened and closed by rotating around an axis L5 extending in the up-down direction (the Z-direction), for example, is provided on a front surface of the upper housing 5a. A display 5b is provided on the front panel 5c. The display 5b is directed forward and displays information necessary for a test to an operator of the urine test device 1. The display 5b may have a touch panel function. In this case, the display 5b can also serve as an operation unit operated by the operator.

The placement table 3 has substantially the same width as the device main body 2 in the left-right direction. The placement table 3 has a predetermined size in plan view. A plurality of racks R can be placed on the placement table 3. Although only one rack R is shown in Fig. 1, a plurality of racks R can be placed on a right side and a left side of the placement table 3. The placement table 3 is provided with a conveying mechanism (not shown) that conveys the rack R in a necessary direction. As a specific configuration of the conveying mechanism, a known configuration can be adopted. As an example, devices (structures) described in Japanese Patent Application Laid-Open Publication No. H10-120167 and Japanese Patent Application Laid-Open Publication No. H10-139152 may be applied.

Returning to Fig. 2, in the device main body 2, for example, a controller 90 is provided in the upper housing 5a. The controller 90 is, for example, a computer including a read only memory (ROM) in which a program or the like is stored, a storage medium such as a random access memory (RAM) in which data is temporarily stored, and a processor such as a central processing unit (CPU). The controller 90 controls operations of a mechanism and a device in the urine test device 1 in accordance with a program stored in advance in response to an operation by the operator, and performs various operations related to the test. The controller 90 can communicate information with various motors and centers included in the supply mechanism 40, a first conveying mechanism 10, and a second conveying mechanism 20, a camera 32, an illuminator 33, and the like of an imaging device 30.

As shown in Figs. 1 and 2, the supply mechanism 40 is a feeder for supplying the test papers C one by one in a predetermined direction (posture). The supply mechanism 40 includes a rectangular parallelepiped housing 40a, a casing 43 disposed inside the housing 40a, and a pair of input lids 41 that are openable and closable with respect to the casing 43. The supply mechanism 40 accommodates therein a plurality of test papers C input by the operator through the input lids 41. The plurality of test papers C are put in a state where directions in a length direction are aligned (in directions shown in Fig. 3) such that portions in which the plurality of reagent parts Ca (see Fig. 3) are arranged at regular intervals are disposed in front. The supply mechanism 40 sequentially discharges the test papers C one by one from a discharge unit 49. In the supply mechanism 40, a sensor (not shown) is provided to detect that one test paper C has been successfully taken out. When the controller 90 detects that the test paper C is to be supplied (the test paper C is to be discharged from the discharge unit 49), the controller 90 controls the first conveying mechanism 10 (see Fig. 3) to be described later to move an arm 11 to a position immediately below the discharge unit 49 at a predetermined timing. The test paper C dropped from the discharge unit 49 is placed on a support portion 11a of the arm 11. Accordingly, the supply mechanism 40 sequentially supplies the test papers C one by one to the first conveying mechanism 10.

The casing 43 of the supply mechanism 40 has an airtight structure, and airtightness between the casing 43 and the input lid 41 is also ensured. A path through which a space in the supply mechanism 40 can be exposed to outside air (atmosphere) is only the discharge unit 49 located at a lower end of the supply mechanism 40. It should not be understood that the term "airtight" means only completely (100%) blocking of the outside air. A person skilled in the art would understand that the term "airtight" includes a case where the passage of outside air is slightly allowed.

In the present embodiment, the supply mechanism 40 includes the pair of input lids 41 and can supply two types (many types) of test papers C. This is merely an example, and the supply mechanism 40 may be of a type capable of supplying only one type of test paper C.

Next, various configurations provided in the device main body 2 will be described with reference to Figs. 2 to 4. The device main body 2 includes the first conveying mechanism 10 that conveys the test paper C received from the supply mechanism 40 to a deposition position Pa or the vicinity thereof, a deposition device 9 that deposits a sample on each reagent part Ca of the test paper C disposed at the deposition position Pa, the second conveying mechanism 20 that conveys the test paper C from the deposition position Pa to an imaging position Pb, and the imaging device 30 that images the test paper C disposed at the imaging position Pb. That is, the device main body 2 deposits a sample on the test paper C and analyzes a specific component in the sample.

The device main body 2 further includes a disposal box 60 that stores the test paper C discharged from the imaging device 30 after the imaging device 30 has finished imaging. The rectangular parallelepiped disposal box 60 is open upward, and can store a large number of (a certain number of) test papers C in a storage space 65 inside. In the present embodiment, for example, an internal space S (see Fig. 1) having a size (a volume) equal to or larger than that of the disposal box 60 is formed below the disposal box 60.

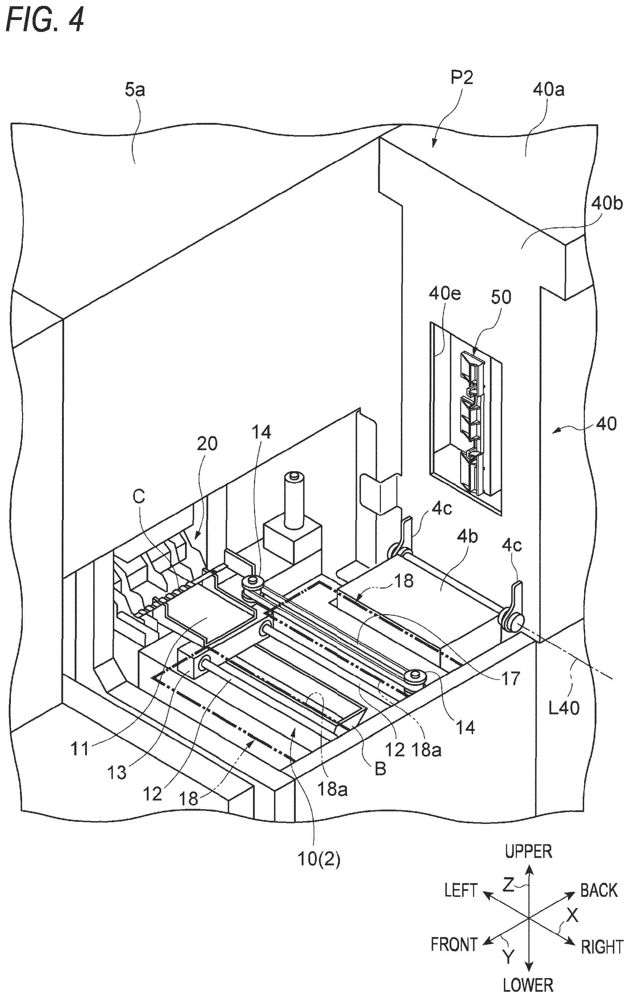

As shown in Figs. 3 and 4, the first conveying mechanism 10 includes, for example, the arm 11 that supports the test paper C by the support portion 11a, a moving block 13 to which the arm 11 is fixed, and a pair of shafts 12 that extend in the X-direction (a horizontal direction) and guide the moving block 13 in the X-direction. The first conveying mechanism 10 includes a drive motor (not shown since the drive motor is hidden under the first conveying mechanism 10) that is controlled by the controller 90, a drive pulley 14 that is rotationally driven by the drive motor, and a timing belt 17 that extends between the drive pulley 14 and a driven pulley 14. The moving block 13 is connected to the timing belt 17. The first conveying mechanism 10 moves the arm 11 to a position directly below the discharge unit 49 at the predetermined timing. The first conveying mechanism 10 receives one test paper C from the supply mechanism 40, and then conveys the test paper C to a predetermined position above the deposition position Pa where the sample is deposited. That is, in the present embodiment, the first conveying mechanism 10 conveys the test paper C received from the supply mechanism 40 to the vicinity of the deposition position Pa.

A rear end of the test paper C conveyed above the deposition position Pa is aligned in a front direction with an extruded plate 15A movable in the front-rear direction. The movement of the extruded plate 15A is adjusted by converting a rotational driving force of an extrusion motor 15B into a straight line by a crank mechanism (not shown). Other known configurations may be appropriately adopted for a mechanism for driving the extruded plate 15A.

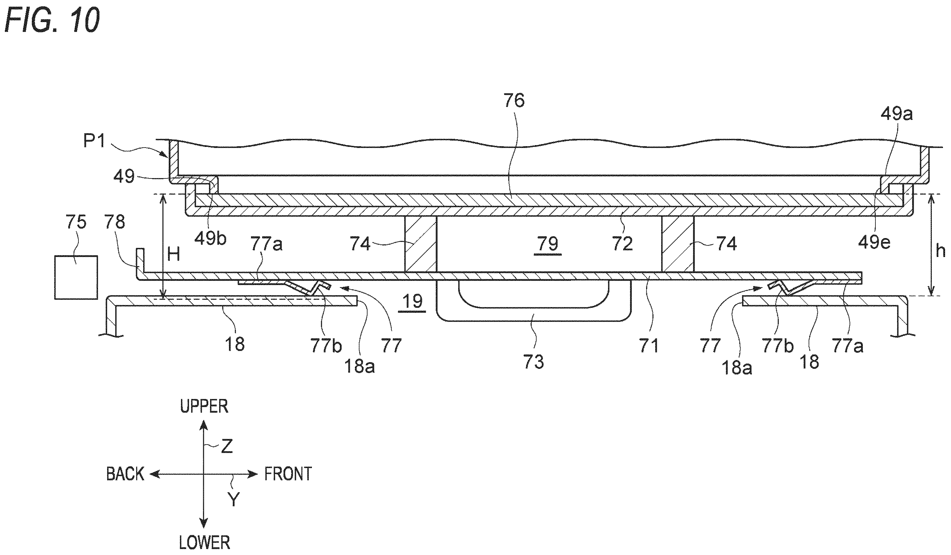

A tray-shaped cleaning tray B having a short side extending in the front-rear direction and a long side extending in the left-right direction is disposed below the pair of shafts 12. The cleaning tray B stores, for example, a powdery reagent scattered from the reagent parts Ca of the test paper C, other dust, and the like. As shown in Fig. 4, the first conveying mechanism 10 includes a pair of top plates 18 that extend directly above (immediately above) the pair of shafts 12 to cover the pair of shafts 12. The pair of top plates 18 extend in the horizontal direction (parallel to an XY plane), and prevent the powdery reagent scattered from the reagent part Ca from being applied to the pair of shafts 12. The pair of top plates 18 are separated from each other in the front-rear direction. Inner ends 18a of the top plates 18 are separated from each other in the front-rear direction, and an open portion 19 (see Fig. 10) is formed between the inner ends 18a. The top plate 18 and the cleaning tray B are main cleaning target portions of the first conveying mechanism 10.

The first conveying mechanism 10 is not limited to the above configuration. The first conveying mechanism 10 may convey, to the deposition position Pa, the test paper C received from the supply mechanism 40. The first conveying mechanism 10 may be any mechanism that can receive the test paper C from the supply mechanism 40 and convey the test paper C in a lateral direction. Other known configurations may be adopted as the first conveying mechanism 10. For example, the first conveying mechanism 10 may include a belt conveyor or the like. The first conveying mechanism 10 may include a slide rail, a linear guide, or the like.

As shown in Figs. 2 and 3, the deposition device 9 collects (samples) a predetermined amount of the sample in the spit 100, and deposits the sample on each reagent part Ca of the test paper C disposed at the deposition position Pa. The deposition device 9 includes a support drive mechanism 9c and a rail 9b disposed in the upper housing 5a, and a nozzle 9a movable in the up-down direction (the Z-direction) and the front-rear direction (the Y-direction) along the rail 9b. The support drive mechanism 9c includes a drive motor (not shown). When the controller 90 detects that the spit 100 arrives at a predetermined position in front of the deposition device 9 (in front of the deposition position Pa), the deposition device 9 is controlled by the controller 90, and the sample is collected by the nozzle 9a. When the nozzle 9a moves forward, the nozzle 9a reaches a position protruding from the device main body 2 (that is, a region of the placement table 3). That is, the nozzle 9a is movable between the device main body 2 and the placement table 3. When the sample is collected, a pump unit PU disposed in the upper housing 5a is also controlled by the controller 90 and operates in conjunction with the deposition device 9.

The deposition position Pa is set at a position slightly higher than an upper end of the spit 100 on the rack R. Accordingly, a stroke in the up-down direction of the nozzle 9a is a minimum distance. That is, a tip 9t of the nozzle 9a needs to enter a predetermined depth in the spit 100, but after the sample is collected, the tip 9t separates from the spit 100 and moves backward at a position slightly higher than an upper end thereof. Thereafter, for example, deposition is performed on each reagent part Ca in order from the rear reagent part Ca. The deposition device 9 repeats the same operation every time a new sample (the spit 100) arrives at the predetermined position. Although not shown, the deposition device 9 includes a cleaning unit or the like that cleans the nozzle 9a between the deposition position Pa and the spit 100.

As shown in Figs. 2 and 3, the second conveying mechanism 20 is, for example, a mechanism that conveys the plurality of test papers C stepwise at a predetermined pitch. In the present embodiment, the imaging position Pb is located on the left of the deposition position Pa and is high. The second conveying mechanism 20 conveys the test paper C obliquely upward. The second conveying mechanism 20 includes, for example, a pair of outer fixing plates 21, a single central fixing plate 22 disposed at an intermediate position between the pair of outer fixing plates 21 in the front-rear direction, and a pair of movable plates 23 disposed between the pair of outer fixing plates 21 and the central fixing plate 22 (since Fig. 3 is a cross-sectional view, the front outer fixing plate 21 is not shown). The outer fixing plates 21, the central fixing plate 22, and the movable plates 23 are disposed parallel to an XZ plane and form a substantially congruent step shape. In the outer fixing plates 21, the central fixing plate 22, and the movable plates 23, all the horizontal step portions are set to have the same pitch (a height difference and a distance in the left-right direction).

The pair of movable plates 23 are moved by a drive motor (not shown) and a rectangular cam to draw a predetermined circulating orbit. More specifically, the pair of movable plates 23 move along a substantially rectangular circulating orbit (a counterclockwise orbit when viewed from the front) so as to move between two adjacent horizontal step portions in the outer fixing plates 21 and the central fixing plate 22. As a result, the second conveying mechanism 20 conveys the test paper C placed on a horizontal step portion to a horizontal step portion immediately above, and repeats the operation.

The test paper C conveyed by the first conveying mechanism 10 above the deposition position Pa is conveyed from the arm 11 of the first conveying mechanism 10 to the deposition position Pa by an operation of the movable plate 23. That is, the test paper C conveyed to the delivery position is lifted upward from the arm 11 by the movable plate 23. At this time, the arm 11 moves to a position immediately below the discharge unit 49 (for conveying the next test paper C). The test paper C lifted upward is placed on the lowest horizontal step portion in the outer fixing plates 21 and the central fixing plate 22, which are the deposition positions Pa, while drawing a substantially rectangular circulating orbit (a clockwise orbit when viewed from the front). The uppermost horizontal step portion in the outer fixing plates 21 and the central fixing plate 22 is disposed inside the imaging device 30 and is the imaging position Pb. Conveyance of the second conveying mechanism 20 is performed in consideration of a time required for color development of the reagent part Ca.

The second conveying mechanism 20 is not limited to the above configuration. The second conveying mechanism 20 only needs to convey the test paper C disposed at the deposition position Pa obliquely upward. Other known configurations may be adopted as the second conveying mechanism 20. For example, the second conveying mechanism 20 may include a belt conveyor or the like. Further, the second conveying mechanism 20 may be capable of conveying the test paper C in the horizontal direction such as a left side. In this case, the imaging position Pb may be at the same height as the deposition position Pa.

As shown in Fig. 1, the imaging device 30 includes an imaging chamber 31 having a rectangular parallelepiped shape (or a cubic shape) and disposed to surround the imaging position Pb, the camera 32 disposed above the imaging chamber 31, and the illuminator 33 disposed on the imaging chamber 31 and around the camera 32. The imaging device 30 reads optical information on the test paper C on which the sample is deposited. The imaging position Pb is located substantially at a center of the imaging chamber 31 and at a bottom of the imaging chamber 31. The test paper C is supported by the arm 11 of the first conveying mechanism 10, and maintains a posture in which a length direction of the test paper C is oriented in the front-rear direction (a direction perpendicular to the plane of Fig. 1) and the reagent part Ca faces upward until the test paper C reaches the imaging position Pb by being conveyed by the second conveying mechanism 20. The camera 32 images the entire test paper C and transmits image information to the controller 90.

The controller 90 performs a test relating to each test item, that is, measurement and determination based on the image of the test paper C acquired by the imaging device 30. The controller 90 causes the display 5b to display a measurement result and a determination result together with unique information such as a sample ID. The controller 90 stores the measurement result and the determination result in a storage unit together with unique information such as the sample ID. The controller 90 has a function of a test unit in the urine test device 1.

The test paper C that has been imaged by the imaging device 30 is discharged from the imaging device 30 by the second conveying mechanism 20, and is put into the disposal box 60.

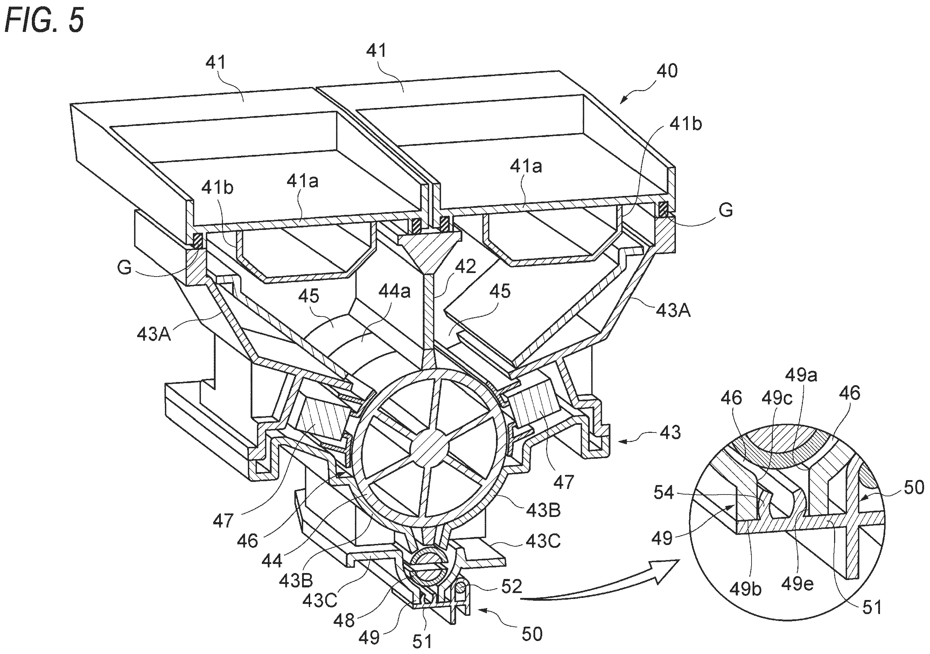

Next, the supply mechanism 40 according to the present embodiment and a configuration related to moisture prevention in the supply mechanism 40 will be described with reference to Fig. 4 and subsequent drawings. Hereinafter, the description of a direction and a positional relation of each part of the supply mechanism 40 is based on a case where the supply mechanism 40 is located at an operating position P1 (to be described in detail later) unless otherwise specified. As shown in Fig. 5, the supply mechanism 40 includes the pair of input lids 41 that seal an open portion on an upper surface of the casing 43, a drum 44 disposed in the casing 43 and rotatable about an axis extending in the front-rear direction (Y-direction), an auxiliary drum 48 disposed below the drum 44, and a discharge unit 49 formed below the auxiliary drum 48 and having an elongated hollow rectangular body shape (a rectangular tubular shape). The discharge unit 49 is formed in an elongated rectangular shape extending in the front-rear direction and slightly larger than the test paper C. An upper-end opening 49c connected to a take-out unit 46 to be described later is formed in an upper-end surface 49a of the discharge unit 49, and a lower-end opening 49e that connects to the outside of the supply mechanism 40 is formed on a lower-end surface 49b of the discharge unit 49. The casing 43 includes an upper casing 43A, a middle casing 43B, and a lower casing 43C. In a state where the input lids 41 close an upper surface of the upper casing 43A, a portion other than the lower-end opening 49e of the discharge unit 49 maintains an airtight structure. An accommodating chamber 45 capable of accommodating the plurality of test papers C is formed between the upper casing 43A, the input lid 41, and an outer peripheral surface 44a of the drum 44.

When the supply mechanism 40 can supply two types (many types) of test papers C, a central partition wall 42 extending in the up-down direction is provided at a central position in the left-right direction. The central partition wall 42 defines two accommodating chambers 45. The input lid 41 includes a flat plate portion 41a and a desiccant accommodating portion 41b integrally provided on a lower surface of the flat plate portion 41a. The desiccant accommodating portion 41b is disposed in the accommodating chamber 45. A plurality of through holes (or slits or the like) are formed in the desiccant accommodating portion 41b, and the inside of the casing 43 is dehumidified by a desiccant (not shown) provided in the desiccant accommodating portion 41b. In addition, a gasket G is provided between the flat plate portion 41a of the input lid 41 and the upper casing 43A in order to improve airtightness.

A groove (not shown) into which the test paper C is fitted is formed in the outer peripheral surface 44a of the drum 44. A depth of the groove is set such that only one test paper C is fitted in the groove, and two or more test papers C cannot be accommodated in the groove. A slight cylindrical clearance is formed between the drum 44 and the middle casing 43B. The drum 44 is rotated clockwise and counterclockwise by a motor (not shown) as viewed from the front. The drum 44 is also called an inversion drum. By the above-described structure and the rotation of the drum 44, one test paper of the plurality of test papers C is taken out from the accommodating chamber 45. The drum 44 and a part of the casing 43 constitute the take-out unit 46 that takes out one test paper C from the accommodating chamber 45.

A pair of front and back detection units 47 are provided on left and right sides of the drum 44. The front and back detection units 47 detect whether the test paper C accommodated in the groove of the drum 44 is facing up or facing down. The test paper C conveyed downward by the drum 44 drops through the lower end opening and is accommodated in a slit portion formed in the auxiliary drum 48. A rotation angle of the auxiliary drum 48 is controlled according to a detection result by the front and back detection units 47, and the test paper C is discharged from the lower-end opening 49e of the discharge unit 49 in a predetermined posture. Accordingly, the dropped test paper C is placed on the arm 11 (see Figs. 3 and 4) of the first conveying mechanism 10 with a front surface facing upward.

As a specific configuration of the supply mechanism 40, another known configuration may be adopted. As an example, a device (a structure) described in Japanese Patent Application Laid-Open Publication No. 2000-35433 may be applied. Alternatively, for example, a cartridge type mechanism may be adopted as the supply mechanism 40.

As shown in Fig. 4, the supply mechanism 40 is attached to the device main body 2 via a base portion 4b and a pair of connectors 4c provided in the device lower portion 4. The supply mechanism 40 is openable and closable with respect to the device main body 2 for maintenance, for example. The supply mechanism 40 is rotatable about a rotation axis L40 extending in the left-right direction (that is, horizontal). A rectangular opening 40e is formed in a central portion of a bottom surface portion 40b of the housing 40a, and the discharge unit 49 is disposed to face the opening 40e. The supply mechanism 40 is movable between the operating position P1 (see Fig. 1) where the discharge unit 49 faces the first conveying mechanism 10 and a maintenance position P2 (see Fig. 4) where the discharge unit 49 is separated from the first conveying mechanism 10 as compared with the operating position P1. It can be said that the operating position P1 is a closed position of the supply mechanism 40, and the maintenance position P2 is an open position of the supply mechanism 40. The maintenance position P2 is a position where the supply mechanism 40 is rotated by about 90 degrees or more from the operating position P1.

The supply mechanism 40 maintains a substantially horizontal posture at the operating position P1. That is, a central axis of the drum 44 extends substantially horizontally (in the Y-direction). The supply mechanism 40 is fixed and set at the operating position P1 by a mechanism such as a ball catch (not shown). A lock mechanism may include a latch or the like. Further, the supply mechanism 40 can maintain its posture even at the maintenance position P2. For example, the supply mechanism 40 does not rotate to a rear side by 90 degrees or more with the engagement of plate members provided in the upper housing 5a and the housing 40a.

When the supply mechanism 40 is set at the operating position P1, the discharge unit 49 is located at a position facing the cleaning tray B via the open portion 19 (see Fig. 10). When the supply mechanism 40 is opened to the maintenance position P2, the cleaning tray B disposed below the first conveying mechanism 10 is exposed. The operator can clean the cleaning tray B and the periphery of the first conveying mechanism 10 when the urine test device 1 is idle.

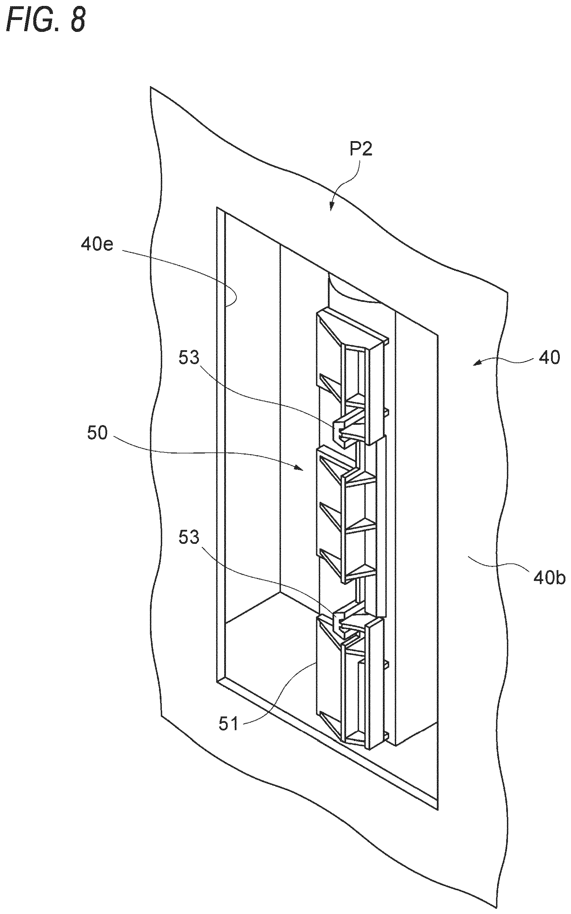

As shown in Figs. 4 and 5, the supply mechanism 40 includes a shutter 50 that is attached to the vicinity of the discharge unit 49 and covers the discharge unit 49. During the operation of the supply mechanism 40, the shutter 50 opens the discharge unit 49 only when the test paper C is discharged, and covers the discharge unit 49 except when the test paper C is discharged. The shutter 50 includes a shutter body 51 rotatably attached to the lower casing 43C via a shaft 52, and a torsion spring (a spring member) (not shown) that biases the shutter body 51 in a closing direction (clockwise when viewed from the front). A pair of engagement projections 53 (see Fig. 8) that engage with the support portion 11a of the arm 11 that moves in a right direction are provided on an outer surface side of the shutter body 51. When the engagement projections 53 are pressed by the support portion 11a, the shutter 50 moves in an opening direction, and the test paper C is discharged. A pair of holding claws 54 are provided on an inner surface side of the shutter body 51 to restrict a posture of the test paper C when discharged. The test paper C is vertically fitted to the holding claws 54 fitted in the discharge unit 49.

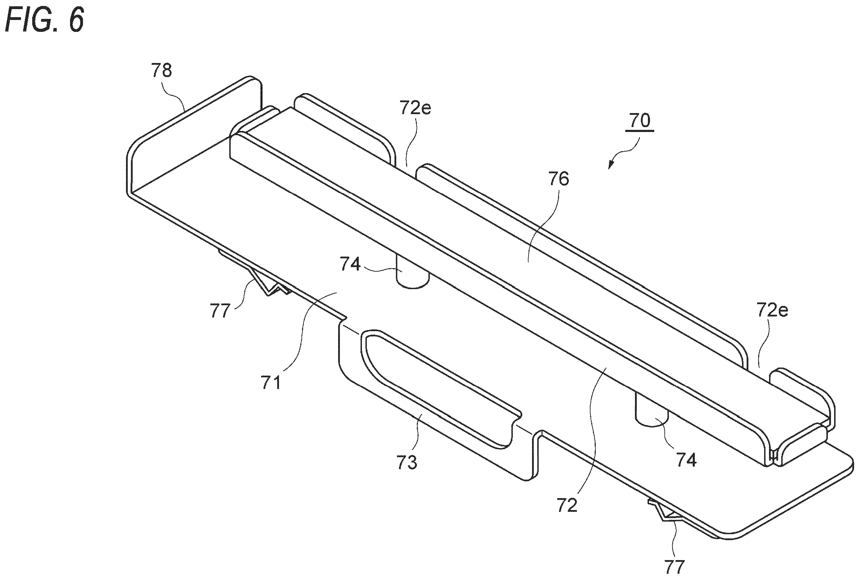

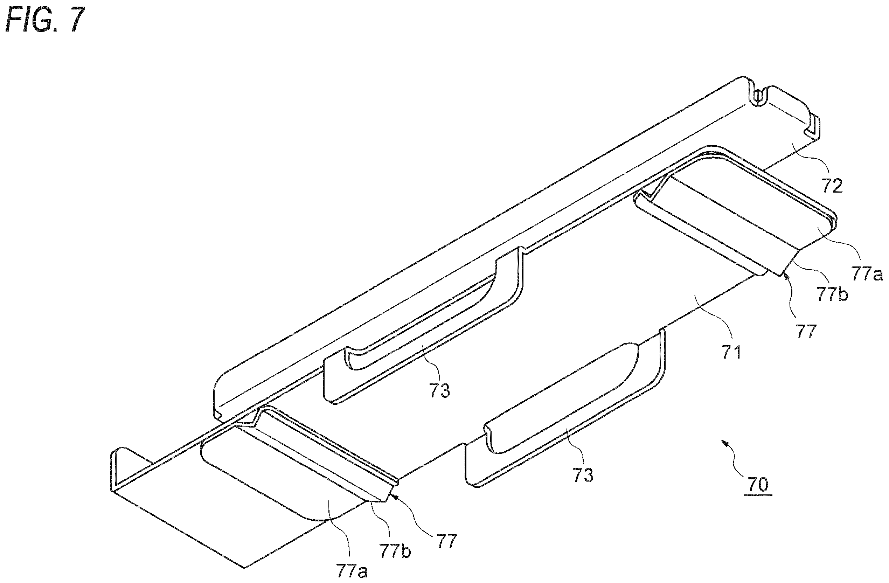

As shown in Figs. 6,7, 9A, and 9B, the urine test device 1 includes a cover 70 for moisture proof inside the supply mechanism 40. The cover 70 is provided to cover the lower-end opening 49e of the discharge unit 49 when the supply mechanism 40 is idle (that is, when the urine test device 1 is idle), and seals the accommodating chamber 45 and the take-out unit 46 against the outside air.

The cover 70 includes a flat plate portion 71 and a sheet accommodating body 72 made of metal such as stainless steel. The flat plate portion 71 has a rectangular shape larger than the discharge unit 49. A rectangular sheet 76 made of a material capable of shielding (preventing permeation) water (moisture) in the outside air is fitted into the sheet accommodating body 72. The sheet accommodating body 72 has an elongated tray-shape smaller than the flat plate portion 71 but slightly larger than the discharge unit 49. The sheet 76 is accommodated in the sheet accommodating body 72 and is fixed by, for example, adhesion. The sheet 76 is larger than the discharge unit 49 and has a size and a shape capable of covering the entire discharge unit 49. The sheet 76 may have elasticity. As the sheet 76, for example, a silicon sheet is used. A predetermined gap 79 (see Fig. 10) is formed between the flat plate portion 71 and the sheet accommodating body 72. The flat plate portion 71 and the sheet accommodating body 72 are connected and fixed to each other by two pillars 74 in a state where the gap is maintained.

A pair of handle portions 73 connected to a pair of long-side portions of the flat plate portion 71 and a pair of plate springs 77 spaced apart from each other in a long-side direction of the flat plate portion 71 are provided on a surface of the flat plate portion 71 opposite to the sheet accommodating body 72. The handle portions 73 are held by one hand of the operator. The plate spring 77 includes a flat plate-shaped base portion 77a fixed to the flat plate portion 71, and a spring portion 77b that rises in a V-shape from the base portion 77a and is displaceable with respect to the flat plate portion 71. On the other hand, one short side portion of the flat plate portion 71 is provided with a detected portion 78 which is a plate piece extending perpendicularly to the flat plate portion 71.

A pair of notches 72e spaced apart from each other in the long-side direction are formed in a side wall provided in a long-side portion of the sheet accommodating body 72.

A method of attaching the cover 70 will be described with reference to Figs. 8 to 10. The cover 70 is attached to the discharge unit 49 when the supply mechanism 40 is located at the maintenance position P2. As shown in Fig. 8, the operator first rotates the supply mechanism 40 to the maintenance position P2 while the supply mechanism 40 is idle. The discharge unit 49 is covered with a shutter 50. The cover 70 is attached to the discharge unit 49 using the shutter 50.

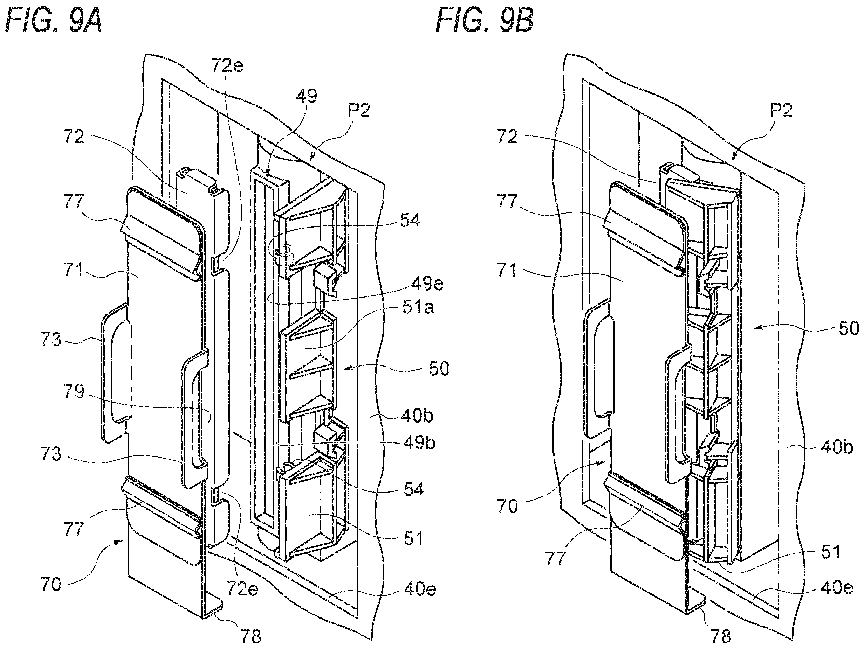

As shown in Fig. 9A, the operator holds the handle portion 73 of the cover 70 with one hand (for example, a left hand), and opens the shutter body 51 with the other hand (for example, a right hand) against a biasing force of the torsion spring. At this time, as the shutter body 51 rotates, the pair of holding claws 54 fitted into the discharge unit 49 are separated from the discharge unit 49. The operator applies the sheet accommodating body 72 (a surface on which the sheet 76 is provided) of the cover 70 to the discharge unit 49 having a rectangular frame shape. Thereafter, as shown in Fig. 9B, the operator tilts the shutter body 51 in a closing direction. A tip portion 51a of the shutter body 51 enters the gap 79 between the sheet accommodating body 72 and the flat plate portion 71. At the same time, the pair of notches 72e allows the pair of holding claws 54 to pass therethrough. The biasing force of the torsion spring is applied, and the sheet accommodating body 72 is pressed by the tip portion 51a. Since the tip portion 51a extends in the long-side direction except for a cutout portion of the shutter body 51, the cover 70 is pressed by the shutter body 51 via a linear contact portion having a predetermined distance.

In a state shown in Fig. 9B, the cover 70 is temporarily held by the discharge unit 49. The cover 70 is temporarily held by the shutter 50. That is, the cover 70 is temporarily held by the discharge unit 49 when the supply mechanism 40 is located at the maintenance position P2. After the cover 70 is temporarily held, the cover 70 does not fall off even when the operator releases one hand from the cover 70. Unless the shutter body 51 is moved in the opening direction again, a temporary holding state of the cover 70 is maintained. Accordingly, even when the operator releases both hands from the cover 70 and the shutter 50 and rotates the supply mechanism 40 toward the operating position P1, the cover 70 does not fall off and maintains a posture of being sandwiched between the discharge unit 49 and the shutter 50. In the temporarily held state, the cover 70 is not fixed and can slightly move with respect to the discharge unit 49 and the supply mechanism 40.

During a rest period of the supply mechanism 40, the supply mechanism 40 is moved to the operating position P1 and set in the device main body 2. When the supply mechanism 40 is set in the device main body 2, the cover 70 is brought into close contact and fixed with the discharge unit 49 along with the setting operation. More specifically, as shown in Fig. 10, when the supply mechanism 40 reaches the operating position P1 and is locked, the lower-end surface 49b of the discharge unit 49 comes into close contact with the sheet 76 so as to close the lower-end opening 49e, and the pair of plate springs 77 come into contact with the pair of top plates 18 of the first conveying mechanism 10. Since an entire height H from a surface of the sheet 76 to an apex of the spring portion 77b when the spring portion 77b is in a free state (an initial state) is smaller than a gap height h from the surface of the sheet 76 to the top plate 18, the spring portion 77b contracts (deforms). The sheet 76 is also compressed (deformed). Accordingly, the cover 70 comes into close contact with the discharge unit 49 by being sandwiched between the discharge unit 49 and the top plate 18 (a portion of the first conveying mechanism 10). In Fig. 10, since the spring portion 77b is in a compressed state, a reference line on a lower side of the entire height H (indicating an apex position of the spring portion 77b in a free state) is shown within a range of a thickness of the top plate 18.

In a state where the cover 70 is attached, the sheet accommodating body 72 is accommodated in the housing 40a (inside the bottom surface portion 40b), and the flat plate portion 71 protrudes outside the housing 40a (see Fig. 9B). The flat plate portion 71 and the handle portion 73 are separated from the sheet accommodating body 72 including the sheet 76. The cover 70 has a larger dimension than the shutter 50 in a passing direction (a thickness direction of the bottom surface portion 40b) of the test paper C in the discharge unit 49. Accordingly, the plate springs 77 are contactable with the top plates 18 without being affected by the presence of the shutter 50. The handle portions 73 are provided to protrude from the flat plate portion 71, and are disposed in the open portion 19 between the pair of inner ends 18a and does not interfere with any member of the first conveying mechanism 10.

A sensor 75 that detects the presence of the cover 70 is attached near the rear top plate 18 (the vicinity of the base portion 4b. See Fig. 4). In a state where the supply mechanism 40 is fixed at the operating position P1 and the cover 70 is fixed, the sensor 75 located behind the detected portion 78 detects the presence of the detected portion 78 attached to the discharge unit 49. For example, if the cover 70 remains attached when the urine test device 1 stands up (starts operating), the controller 90 receives a signal from the sensor 75 and issues a warning through the display 5b or another notification unit (alarm or the like). When the cover 70 remains attached to the discharge unit 49, the controller 90 does not start the test of the urine test device 1. That is, in this case, the controller 90 performs operation prohibition control to prohibit the operation of the urine test device 1 and issues a warning. The operation prohibition control can prevent the supply mechanism 40 and the first conveying mechanism 10 from operating while the cover 70 is erroneously attached.

According to the urine test device 1 in the present embodiment, while the supply mechanism 40 is operating, the test papers C are discharged one by one from the discharge unit 49. The test paper C is conveyed to the vicinity of the deposition position Pa by the conveying mechanism 400. After the sample (that is, urine) is deposited on the reagent part 201 of the test paper C, a test relating to the sample is performed. When the supply mechanism 40 is idle, for example, when the device is not operated for a while after the test of a large number of samples is finished, such as when the measurement of one day is finished, the discharge unit 49 can be covered with the cover 70. Since the cover 70 seals the accommodating chamber 45 and the take-out unit 46 against the outside air, even in a state where one or a large number of test papers C are accommodated in the accommodating chamber 45, it is possible to prevent moisture from entering and prevent deterioration of the reagent part 201. Since the cover 70 may be designed in accordance with a shape of the discharge unit 49 or the like, large modification or the like of an existing device is unnecessary. Thus, according to the urine test device 1, it is possible to prevent deterioration of the reagent part 201 due to moisture in the test paper C with a simple configuration that does not require large modification of an existing device. In the case of a device that does not include the cover 70 in the related art, in order to prevent deterioration of the reagent part due to moisture, it is necessary to take measures such as taking out all the test papers C remaining in the accommodating chamber 45 and separately storing the test papers C. In the urine test device 1, since the cover 70 seals the inside of the supply mechanism 40, such special measures are unnecessary.

The cover 70 is attached to the discharge unit 49 when the supply mechanism 40 is located at the maintenance position P2. When the supply mechanism 40 is idle (when the test is finished or the like), the supply mechanism 40 is often moved to the maintenance position P2 for cleaning work or the like. Thus, the cover 70 can be attached using an opportunity or time of the maintenance work. Further, even when the maintenance work is not performed, the discharge unit 49 can be exposed only by moving the supply mechanism 40, and the cover 70 can be easily attached.

Since the cover 70 is brought into close contact and fixed along with the setting operation of the supply mechanism 40, it is possible to reliably and easily attach the cover 70.

There is originally a space between the discharge unit 49 and the first conveying mechanism 10. When the cover 70 is disposed in this space and a pressing force received from the discharge unit 49 and the conveying mechanism 10 is used, a separate pressing mechanism or the like for close contact with the discharge unit 49 (that is, sealing of the supply mechanism 40) is unnecessary.

Further, since the cover 70 is attached using the existing shutter 50, a dedicated fixture, an attachment bracket, or the like is unnecessary. The above-described functions and effects are realized with a simpler configuration.

Although the embodiment of the present disclosure has been described above, the present disclosure is not limited to the above embodiment. For example, a configuration of the cover 70 is not limited to the above-described embodiment, and can be appropriately changed.

The supply mechanism may be detachable from the device main body 2 instead of the rotation type. The supply mechanism may be fixed instead of being openable and closable. In this case, a cover can also be provided between the supply mechanism and the conveying mechanism when the device is idle (namely, at rest).

A structure in which the cover is brought into close contact with the discharge unit based on an attachment structure when the cover is attached without depending on a pressing force from the supply mechanism may be applied. The cover may be attached to any location in the supply mechanism even during operation of the urine test device 1. In this case, the cover is disposed at a place different from the discharge unit 49, and is attached to cover the supply mechanism 40 when the supply mechanism 40 is idle although the discharge of the test paper C from the discharge unit 49 is not hindered.

A cover may be attached without using the shutter 50. The shutter 50 may be omitted from the supply mechanism 40.

## Claims

1. A urine test device comprising:
a conveying mechanism (10) configured to convey a test paper (C) provided with a reagent unit;
a supply mechanism (40) that includes an accommodating chamber capable of accommodating a plurality of test papers (C), a take-out unit (46) configured to take out a test paper (C) from the accommodating chamber and a discharge unit (49) configured to discharge the test paper (C), and that is configured to sequentially supply the test paper (C) to the conveying mechanism (10) by discharging the test paper (C) from the discharge unit (49);
the conveying mechanism (10) configured to receive the test paper (C) from the supply mechanism (40), and convey the test paper (C) to a deposition position (Pa) to which a sample is deposited or a vicinity thereof; and
a cover (70) configured to cover the accommodating chamber and the discharge unit (49), and seal the take-out unit (46) against outside air;
**characterised in that** the urine test device further comprises
a sensor (75) configured to detect presence of the cover (70), wherein
the device main body (2) is provided with a controller (90) configured to control an operation of the urine test device (1),
the controller (90) performs operation prohibition control to prohibit the operation of the urine test device (1) when detecting the cover (70) being attached to the discharge unit (49), and
the controller (90) outputs an alarm in response to the presence of the cover (70) being detected by the sensor (75) when the urine test device (1) starts operating.

2. The urine test device according to claim 1, wherein
the cover (70) is in close contact with the discharge unit (49) by being sandwiched between the discharge unit (49) and a part of the conveying mechanism, when the supply mechanism (40) is idle.

3. The urine test device according to claim 2, further comprising:
a device main body (2) including the conveying mechanism (10), wherein
the supply mechanism (40) is attached to the device main body (2) and is movable between an operating position (P1) where the discharge unit (49) faces the conveying mechanism (10) and a maintenance position (P2) where the discharge unit (49) is separated from the conveying mechanism (10) compared to the operating position (P1), and
the cover (70) is attached to the discharge unit (49) when the supply mechanism (40) is located at the maintenance position (P2).

4. The urine test device according to claim 3, wherein
the cover (70) is temporarily held by the discharge unit (49) when the supply mechanism (40) is located at the maintenance position (P2), and is fixed in close contact with the discharge unit (49) along with a setting operation in which the supply mechanism (40) is moved to the operating position (P1) and set in the device main body (2).

5. The urine test device according to any one of claims 1 to 4, wherein
the supply mechanism (40) includes a shutter (50) attached in the vicinity of the discharge unit (49) and configured to open the discharge unit (49) only at the time of discharging of the test paper (C) and cover (70) the discharge unit (49) at a time other than the time of discharging, and
the cover (70) is attached to the discharge unit (49) using the shutter.

6. The urine test device according to claim 5, wherein
the shutter (50) includes a spring member, and
the cover (70) is attached to the discharge unit (49) by a biasing force of the spring member.

## Patentansprüche

1. Ein Urintestgerät, umfassend
einen Fördermechanismus (10), der so ausgelegt ist, dass er ein mit einer Reagenzeinheit versehenes Testpapier (C) fördert;
einen Zuführmechanismus (40), der eine Aufnahmekammer umfasst, die mehrere Testpapiere (C) aufnehmen kann, eine Entnahmeeinheit (46), die so ausgelegt ist, dass sie ein Testpapier (C) aus der Aufnahmekammer entnimmt und eine Ausgabeeinheit (49), die so ausgelegt ist, dass sie das Testpapier (C) ausgibt, und der so ausgelegt ist, dass er das Testpapier (C) durch Ausgeben des Testpapiers (C) aus der Ausgabeeinheit (49) nacheinander dem Fördermechanismus (10) zuführt;
wobei der Fördermechanismus (10) so ausgelegt ist, dass er das Testpapier (C) vom Zuführmechanismus (40) aufnimmt und das Testpapier (C) zu einer Aufbringposition (Pa), an der eine Probe aufgebracht wird, oder in deren Nähe befördert; und
eine Abdeckung (70), die so ausgelegt ist, dass sie die Aufnahmekammer und die Ausgabeeinheit (49) abdeckt, und die Entnahmeeinheit (46) gegenüber der Außenluft abdichtet;
**dadurch gekennzeichnet, dass** das Urintestgerät ferner folgendes umfasst:
einen Sensor (75), der so ausgelegt ist, dass er das Vorhandensein der Abdeckung (70) erfasst, wobei
der Gerätehauptkörper (2) mit einer Steuereinheit (90) versehen ist, die so ausgelegt ist, dass sie den Betrieb des Urintestgeräts (1) steuert,
die Steuereinheit (90) eine Betriebssperrsteuerung durchführt, um den Betrieb des Urintestgeräts (1) zu unterbinden, wenn sie erkennt, dass die Abdeckung (70) an der Ausgabeeinheit (49) angebracht ist, und
die Steuereinheit (90) einen Alarm ausgibt, wenn der Sensor (75) das Vorhandensein der Abdeckung (70) erfasst, während das Urintestgerät (1) den Betrieb aufnimmt.

2. Urintestgerät nach Anspruch 1, wobei
die Abdeckung (70) in engem Kontakt mit der Ausgabeeinheit (49) steht, indem sie zwischen der Ausgabeeinheit (49) und einem Teil des Fördermechanismus angeordnet ist, wenn der Zuführmechanismus (40) im Leerlauf ist.

3. Urintestgerät nach Anspruch 2, ferner umfassend:
einen Gerätehauptkörper (2), der den Fördermechanismus (10) umfasst, wobei
der Zuführmechanismus (40) am Gerätehauptkörper (2) angebracht und zwischen einer Betriebsposition (P1), in der die Ausgabeeinheit (49) dem Fördermechanismus (10) zugewandt ist, und einer Wartungsposition (P2), in der die Ausgabeeinheit (49) im Vergleich zur Betriebsposition (P1) vom Fördermechanismus (10) getrennt ist, bewegbar ist, und
die Abdeckung (70) an der Ausgabeeinheit (49) angebracht ist, wenn sich der Zuführmechanismus (40) in der Wartungsposition (P2) befindet.

4. Urintestgerät nach Anspruch 3, wobei
die Abdeckung (70) vorübergehend von der Ausgabeeinheit (49) gehalten wird, wenn sich der Zuführmechanismus (40) in der Wartungsposition (P2) befindet, und im engen Kontakt mit der Ausgabeeinheit (49) fixiert wird, und zwar im Zuge eines Einstellvorgangs, bei dem der Zuführmechanismus (40) in die Betriebsposition (P1) bewegt und im Gerätehauptkörper (2) fixiert wird.

5. Urintestgerät nach einem der Ansprüche 1 bis 4, wobei
der Zuführmechanismus (40) eine Klappe (50) umfasst, die in der Nähe der Ausgabeeinheit (49) angebracht und so ausgelegt ist, dass sie die Ausgabeeinheit (49) nur zum Zeitpunkt der Ausgabe des Testpapiers (C) öffnet und die Ausgabeeinheit (49) zu anderen Zeitpunkten als dem Zeitpunkt der Ausgabe abdeckt, und
die Abdeckung (70) unter Verwendung der Klappe an der Ausgabeeinheit (49) angebracht ist.

6. Urintestgerät nach Anspruch 5, wobei
die Klappe (50) ein Federelement umfasst und
die Abdeckung (70) durch eine Vorspannkraft des Federelements an der Ausgabeeinheit (49) angebracht ist.

## Revendications

1. Dispositif d'analyse d'urine comprenant :
un mécanisme de transport (10) configuré pour transporter un papier de test (C) muni d'une unité de réactif ;
un mécanisme d'alimentation (40) qui comprend une chambre de logement capable de loger une pluralité de papiers de test (C), une unité de prélèvement (46) configurée pour prélever un papier de test (C) de la chambre de logement et une unité de sortie (49) configurée pour délivrer le papier de test (C), et qui est configuré pour fournir de manière séquentielle le papier de test (C) au mécanisme de transport (10) en délivrant le papier de test (C) depuis l'unité de sortie (49) ;
le mécanisme de transport (10) étant configuré pour recevoir le papier de test (C) provenant du mécanisme d'alimentation (40), et pour transporter le papier de test (C) vers une position de dépôt (Pa) à laquelle un échantillon est déposé ou à proximité de celle-ci ; et
un couvercle (70) configuré pour recouvrir la chambre de logement et l'unité de sortie (49), et pour isoler hermétiquement l'unité de prélèvement (46) de l'air extérieur ; **caractérisé en ce que** le dispositif d'analyse d'urine comprend en outre
un capteur (75) configuré pour détecter la présence du couvercle (70), dans lequel
le corps principal de dispositif (2) est muni d'un contrôleur (90) configuré pour commander un fonctionnement du dispositif d'analyse d'urine (1),
le contrôleur (90) effectue une commande d'interdiction de fonctionnement afin d'empêcher le fonctionnement du dispositif d'analyse d'urine (1) lorsqu'il détecte que le couvercle (70) est fixé à l'unité de sortie (49), et
le contrôleur (90) émet une alarme en réponse à la détection de la présence du couvercle (70) par le capteur (75) lorsque le dispositif d'analyse d'urine (1) commence à fonctionner.

2. Dispositif d'analyse d'urine selon la revendication 1, dans lequel
le couvercle (70) est en contact étroit avec l'unité de sortie (49) en étant pris en sandwich entre l'unité de sortie (49) et une partie du mécanisme de transport, lorsque le mécanisme d'alimentation (40) est au repos.

3. Dispositif d'analyse d'urine selon la revendication 2, comprenant en outre :
un corps principal de dispositif (2) comprenant le mécanisme de transport (10), dans lequel
le mécanisme d'alimentation (40) est fixé au corps principal de dispositif (2) et est mobile entre une position de fonctionnement (P1), dans laquelle l'unité de sortie (49) fait face au mécanisme de transport (10), et une position d'entretien (P2), dans laquelle l'unité de sortie (49) est séparée du mécanisme de transport (10) comparé à la position de fonctionnement (P1), et
le couvercle (70) est fixé à l'unité de sortie (49) lorsque le mécanisme d'alimentation (40) se trouve en position d'entretien (P2).

4. Dispositif d'analyse d'urine selon la revendication 3, dans lequel
le couvercle (70) est temporairement maintenu par l'unité de sortie (49) lorsque le mécanisme d'alimentation (40) se trouve en position d'entretien (P2), et est fixé en contact étroit avec l'unité de sortie (49) conjointement à une opération de mise en place dans laquelle le mécanisme d'alimentation (40) est déplacé vers la position de fonctionnement (P1) et installé dans le corps principal de dispositif (2).

5. Dispositif d'analyse d'urine selon l'une des revendications 1 à 4, dans lequel
le mécanisme d'alimentation (40) comprend un obturateur (50) fixé à proximité de l'unité de sortie (49) et configuré pour ouvrir l'unité de sortie (49) uniquement au moment de la délivrance du papier de test (C) et pour recouvrir l'unité de sortie (49) à un moment autre que celui de la délivrance, et
le couvercle (70) est fixé à l'unité de sortie (49) au moyen de l'obturateur.

6. Dispositif d'analyse d'urine selon la revendication 5, dans lequel
l'obturateur (50) comprend un élément à ressort, et
le couvercle (70) est fixé à l'unité de sortie (49) par une force de rappel de l'élément à ressort.
